# EUROPEAN PATENT SPECIFICATION

(11) **EP 2 364 658 B1**
(45) Date of publication and mention of the grant of the patent: **29.08.2012**
(21) Application number: 10002468.6
(22) Date of filing: 10.03.2010
(51) Int. Cl.: A61B 17/80, A61B 17/17

(54) **Bone plate system**
Knochenplattesystem
Système de lame osseuse

(43) Date of publication of application: 14.09.2011
(73) Proprietor: aap Implantate AG, 12099 Berlin (DE)
(72) Inventor: Batsch, Thomas, 10367 Berlin (DE); Paulin, Thomas, 14552 Wilhelmshorst OT Michendorf (DE); Fischer, Hans-Joachim, Dr., 12277 Berlin (DE); Alemu, Bruke Seyoum, 14195 Berlin (DE)
(74) Representative: Herden, Andreas F.

(56) References cited:
- WO-A1-01/12081
- WO-A1-2007/079814
- DE-A1- 4 343 117
- US-B1- 6 306 136

## Description

### FIELD OF THE INVENTION

The invention relates to a system for fixating parts of a fractured bone including a bone plate adapted to cooperate with bone screws having a head and a screw shaft for fixating parts of a fractured bone.

### BACKGROUND OF THE INVENTION

Bone plates are known, for example, from CH 462375, WO 2000/53111, WO 2001/54601, EP 0760632 B1 or US 7,229,445 B2.

Such known bone plates include a plurality of holes for cooperation with bone screws, and some of these holes can be shaped as combination holes containing a first hole portion and a second hole portion which overlap and are in communication with one another. The holes are drifted through the bone plate such as to form a contour of two intersecting circles with a pair of tips at the intersecting plane. The first hole portion is of different diameter to the second hole portion, and a screw shaft fitting into the first hole portion, due to the tips between the hole portions, cannot be moved from one hole portion to the other hole portion.

DE 43 43 117 A1 discloses a bone plate comprising an elongated plate defining a longitudinal axis along its length having an upper surface, a lower surface for application to a bone, and a plurality of elongated holes arranged along the length of the plate for receiving bone screws, said elongated holes being oblong in shape and having a major axis D_{L} and a minor axis D_{G}, with D_{L}, being greater than D_{G}, wherein at least one of said elongated holes includes partial threaded portions arranged on an inner wall of the elongated hole, and is configured for seating a bone screw having a threaded head.

The same type of bone plate is disclosed in later filed US 5,709,686 also.

US 7,229,445 B2 shows one hole portion having a thread extending 270 degrees along the hole wall circumference, whereas the other hole portion has smooth wall surfaces on tapering and widening wall portions. A special bone screw type must be used for each hole portion to which it is adapted. Thus, the first hole portion has its own functionality different from the functionality of the second hole portion. There is no functional combination between both hole portions.

WO 2007/079814 A1 = US 2008/0161860 A1 concerns a bone plate with oblong holes which are composed of two intersecting hole portions, however, without tips at the intersecting plane of the hole portions. One hole portion has an upper smooth spherical region and a lower smooth cylindrical region and the other hole portion has an upper threaded conical or spherical region and a lower smooth conical region. Thus, the oblong hole is free from any barrier and can be used in combination of both hole portions for inserting bone screws of different types. The first type is a standard bone screw with a head having a rounded shoulder. The second type is a bone screw with a screw head having a conical or spherical surface with the upper portion thereof carrying a thread and the lower portion thereof being of a smooth bearing surface. The second screw type is for driving the screw perpendicularly to the bone plate plane whereas the standard screw type allows fixation in slanting direction to the bone plate plane in a relatively large angle area. Furthermore, the bone plate can be used to create a relative movement between plate and screw transversely to the driving-in direction of the screw in order to press bone fractures together. However, there is a drawback with bone fixing system of WO 2007/079814 A1, namely hidden gaps. The correct implant position of the bone screw is defined by the cooperation of the smooth conical or spherical bearing surface of the head with the smooth conical or spherical bearing surface in the bone plate which for reasons of manufacture makes it necessary to have a tolerance gap between the thread on the screw head and the thread in the bone plate hole. This tolerance gap between the threads should be as small as possible, so that the bone plate when implanted does not offer hidden gaps wherein liquids from the human body can penetrate. If such liquid solidificates, later removal of the bone plate becomes a problem. However, the threads on the cones of the bone plate and the bone screw in relation to the conical bearing surfaces cannot be produced with narrow tolerances as desired for the purpose intended. Furthermore, the mutual gripping effect of threads on conical surface is rather poor for the sliding-in head thread leaving gaps between the meshing threads.

### SUMMARY OF THE INVENTION

It is an object of the invention to provide a bone fixing system that enables to press together fractured bones.

It is another object of the invention to minimize hidden gaps between bone screw and bone plate implanted.

It is a further object of the invention to make it easier to unthread the bone screws implanted.

The bone plate of the bone fixing system of invention has a plurality of oblong holes where at least one of these oblong holes comprises a first hole region, a second hole region and a transitional region between the first and second regions. The first hole region comprises an upper segment of cylindrical shape and a lower segment of tapering shape. The upper segment includes a thread and the lower segment includes a smooth bearing surface to form a support structure. The second hole region comprises an upper segment of semi-circular cross section and with a smooth bearing surface for offering a support for a screw head having a rounded shoulder. The second hole region also comprises a lower section of cylindrical or widening shape for offering a passage for a bone screw having a screw shaft. The first and second hole regions partially overlap at the transitional region which forms a lateral passage free of any barrier between the lower segments of the first and second hole regions so that portions of the screw shaft of a bone screw can be moved also between the lower segments in order to have the possibility for producing a relative, lateral movement between bone plate and screw in the direction of the oblong hole. For this relative lateral movement, the transitional region is formed reaching into the upper segments of the first and second hole regions and forming a slanting or sloping guideway towards the cylindrically shaped thread in the first hole region. Thus, the cylindrical thread of the bone screw has a tendency to be laterally pressed onto the cylindrical thread in the bone plate hole and minimize the gap between the cylindrical threads of the bone screw and the bone plate.

In a preferred embodiment of the invention, the first hole region which includes the thread on cylindrical surface is of a diameter larger than the second hole region having smooth surfaces. The thread on the first hole region extends on a circumferential angle of between 120 and 180 or less than 180 degrees and preferably close to 175 or 179 degrees.

On the lower side of the bone plate, each oblong hole makes a first opening with a first circular edge, and a second opening with a second circular edge. The first circular edge extends along a circumferential angle of more than 180 degrees, whereas the second circular edge of the second opening does not exceed 180 degrees. The first circumferential angle of the first hole region is between 200 and 300 degrees, whereas the second circumferential angle of the second hole region is between 160 and 180 degrees. When the axis of the first hole region and the axis of the second hole region take a distance such that the edge circles of the holes would produce tips from the overlapping circles, such tips are avoided by tangentially machining the walls from the smaller diameter opening to the larger diameter opening, that is, forming straight wall portions without narrowing the passage from the smaller opening to the bigger opening.

Similar cut-off machining is made when combining the upper segment of the second hole region, which is of generally smaller diameter than the first region, with the upper segment of the first region. Tips are cut-off, and a smooth transition between the upper segment of the second region and the upper segment of the first region is created when machining the slanting guideway towards the cylindrically shaped thread in the first hole region.

In a preferred embodiment, a recess is provided between the upper and the lower segments of the first hole region. This allows the bone screw with the rounded shoulder to enter into this recess with the shoulder rim and make it possible to skew the screw axis to a larger degree relative to the plate plane.

The invention also includes a system for fixating fractured bones comprising a bone plate and bone screws. The bone plate includes oblong holes with hole portions that are free from any barrier for a screw shaft moved between the hole portions. The hole portions also include support structures for the screw heads and a sloping guideway between the hole portions. One of the hole portions has an upper segment with cylindrical thread on an arc of less than 180 degrees, say 175 or 179 degrees and to which the guideway is sloping. An adapted screw with cylindrical thread, when mounted in the bone plate, minimizes the gap between the threads.

### BRIEF DESCRIPTION OF THE DRAWINGS

Additional benefits and features of the invention will emerge from the following description of embodiments by means of the enclosed drawings.

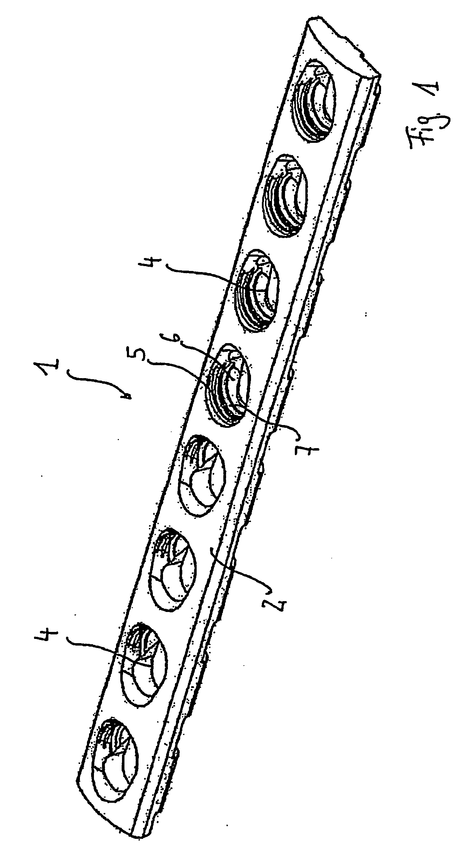

Fig. 1 is a bone plate in a three-dimensional view,
Fig. 2 is an enlarged sectional view from Fig. 1 with a tool for producing a bone plate portion,
Fig. 3 is an enlarged view onto the bottom side of the bone plate from Fig. 1,
Fig. 4 is an enlarged view onto the bottom side of an alternative embodiment of a bone plate,
Fig. 5 is a cross section, in enlarged view, through one oblong hole,
Fig. 6 is a top view onto an oblong hole of a bone plate,
Fig. 7 is a three-dimensional view onto a bone plate portion,
Fig. 8 is an enlarged view onto a bone screw with cylindrical thread on the head,
Fig. 9 is a view onto a bone screw with a rounded shoulder,
Fig. 10 is an enlarged view onto a bone plate with the bone screws of Figs. 8 and 9 inserted therein,
Fig. 11 is a view of the bone screw of Fig. 8 in cooperation with the cylindrical thread and the support structure of a bone plate,
Fig. 12 is a view of the bone screw of Fig. 9 in a skew position in the oblong hole for fixation of the bone plate, and
Fig. 13 is a sequence of three individual views onto a bone plate and a bone screw to illustrate relative movement between bone screw and bone plate for bringing fractured bone portions together.

### DETAILED DESCRIPTION

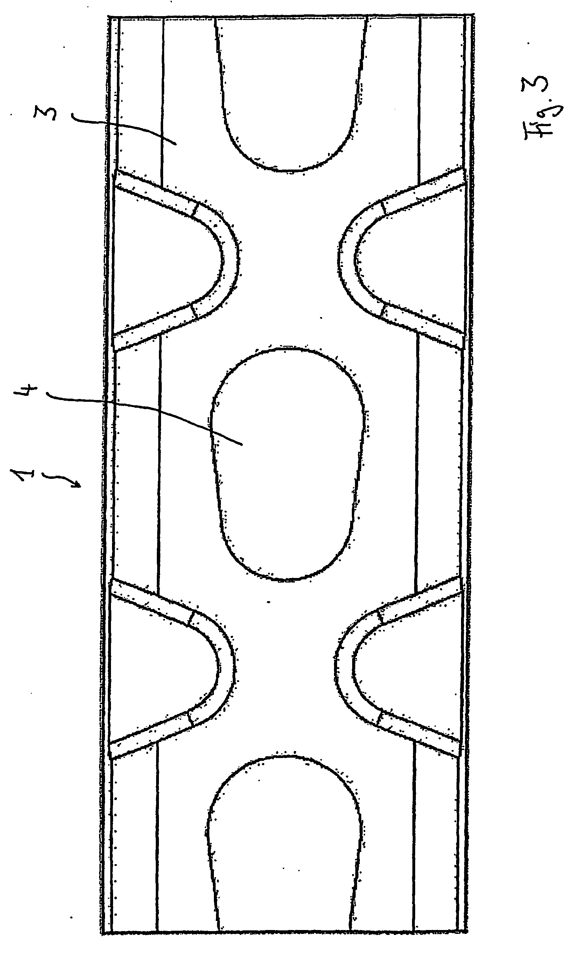

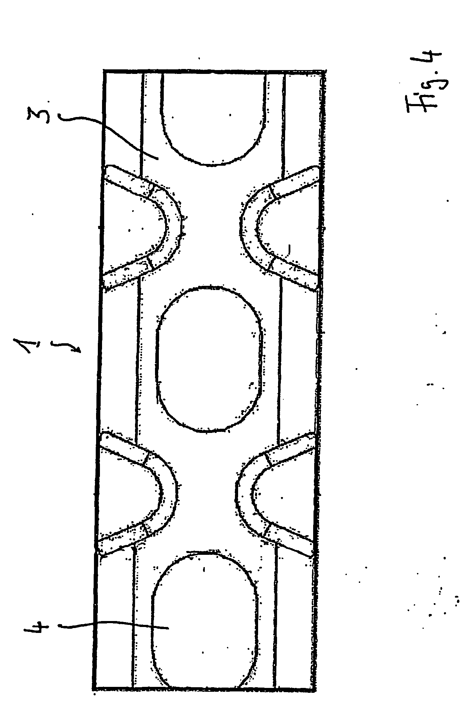

The bone plate of the invention comprises a stripe-formed plate body 1 of tissue tolerance rigid material and defines a body plane and a longitudinal axis along the body. The plate body 1 has an upper side 2 and a lower side 3, serving to bear against the bone being fixated. Continuous openings in the form of oblong holes 4 are introduced into the bone plate between upper side and lower side. The oblong holes 4 are continuous in these embodiments, i. e., configured without any projections or similar obstructions reaching into the interior. The invented bone plate 1 has eight oblong holes 4 overall in these embodiments, yet without being limited to this number. The opening edges of the oblong holes 4 are configured as narrowing toward one or the other end of the plate body 1, as shown in Fig. 3. However, as is shown in an alternative embodiment per Fig. 4, the opening edges can also extend parallel to the longitudinal axis of the plate body 1 and correspond to the shape produced by displacing a circle along a predetermined distance. Of course, all other conceivable shapes of oblong holes are also possible, as long as they are continuous in form, i.e., free of obstruction in their interior.

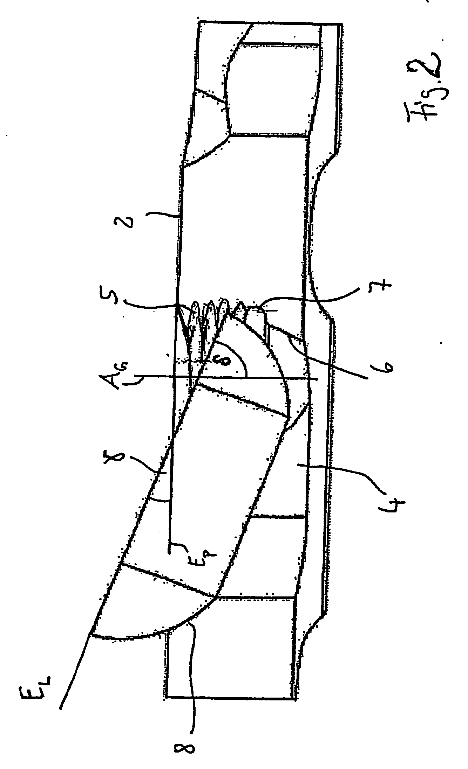

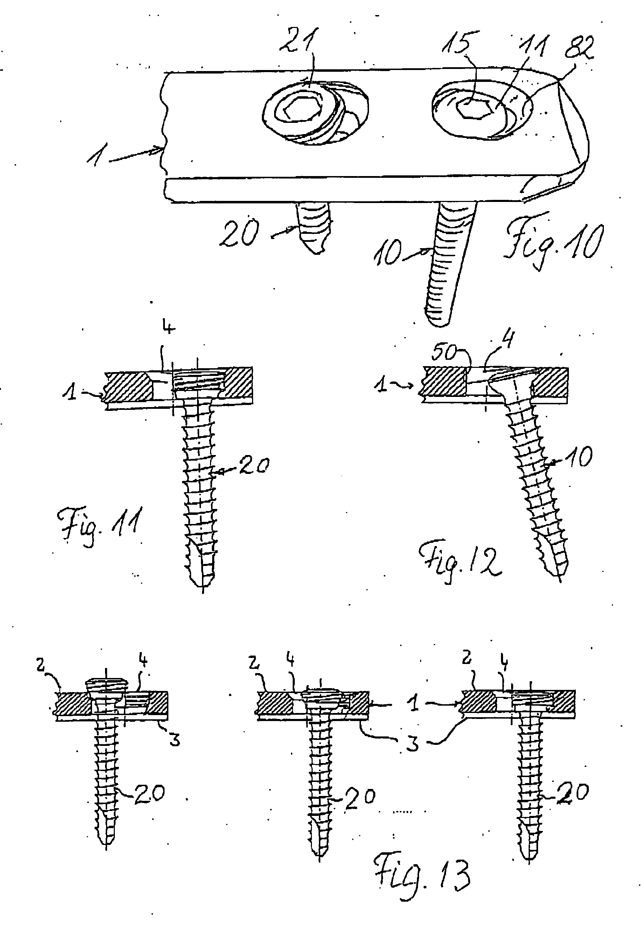

At one end face of each oblong hole are formed a thread or thread flights 5, as well as a support structure 6. The thread 5 and the support structure 6 lie one above the other in a direction transversely to the plane of the plate, namely the thread 5 is arranged on top (toward the upper side 2 of the bone plate 3) and the support structure 6 underneath (toward the lower side 3 of the bone plate 1). Between thread 5 and support structure 6, a recess 7 can be provided (see Fig. 2), However, this recess is not absolutely essential, and it is not present in the embodiments shown in Figures 11 through 13. The support structure 6 is formed by a smooth-walled segment of the oblong hole, being hemispherical or conical.

The thread 5 and support structures 6 of the oblong holes 4 can be arranged at the narrow sides, or the larger sides of the oblong holes 4. The bone plate which is stripe-formed defines a body plane and a longitudinal axis. The oblong holes 4 have longitudinal axes coincident to the longitudinal axis of the bone plate.

The oblong hole 4 contains a circumferential guide structure 8 at its opening facing the upper side 2 of the bone plate 1. A rim is formed with semicircular cross section as a portion of the oblong hole 4 by a chip-removal machining step (such as milling). As an illustration of this, Fig. 2 shows a "bathtub" shaped trend of this rim, i. e., the guide structure 8. One also notices here that the guide structure 8 is inclined at an angle γ to the plane E_{P} of the plate. Likewise, the guideway or guide plane E_{L} is also inclined by an angle δ relative to the axis A_{G} of the thread 5.

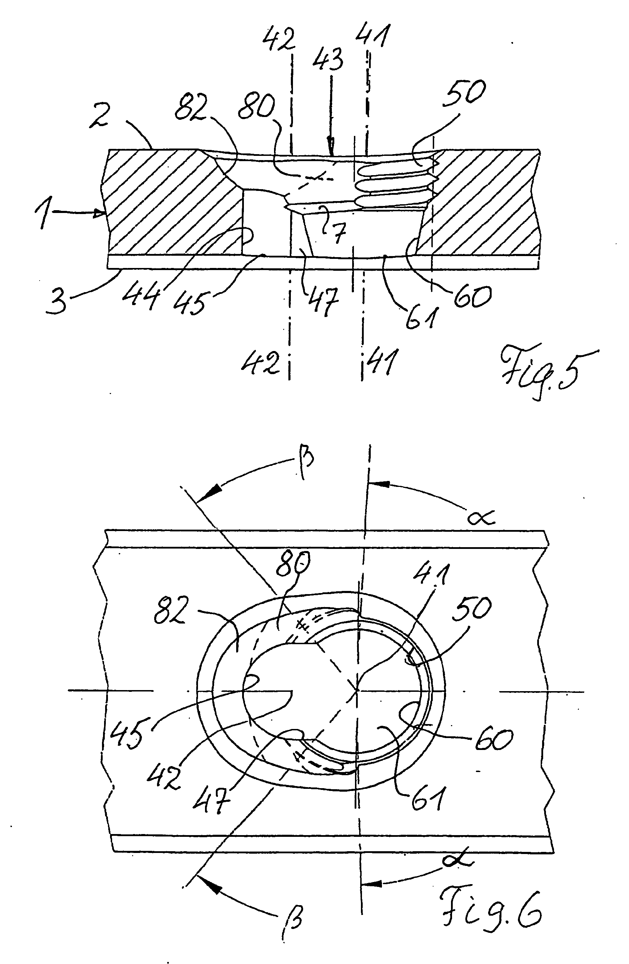

Generally, the thread flights 5 run in a shorter circumferential segment of the oblong hole 4 than the support structure 6 (Fig. 6). While the thread 5 extends over an angle α of between 120 and 180 or less than 180 degrees and preferably close to 175 or 179 degrees, the support structure 6 extends over an angle range β of between 185 and 300 degrees and preferably close to 260 degrees.

This choice of different wrap regions allows for a smooth sliding of the bone screw into this region with a reliable fixation at the end, as shall be further explained below.

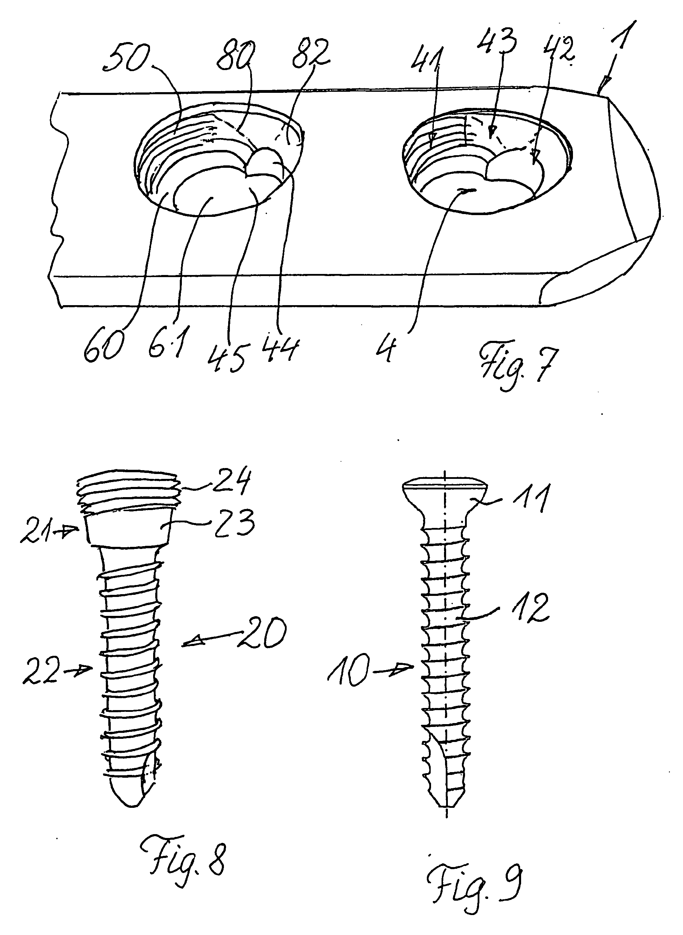

Figure 8 shows a bone screw 20 of the invention. The bone screw 20 includes a head 21 and a screw shaft 22. The head 21 has a tapering portion 23 adjacent to the screw shaft 22, and a cylindrical portion with cylindrical thread 24 thereon, opposite to the screw shaft. The tapering portion 23 complementarily corresponds to the tapering segment 60 of the bone plate and is preferably conical. The screw head 21 has also a hexagon socket (Fig. 10).

Figure 9 shows a standard bone screw 10 and includes a head 11 and a screw shaft 12. The screw head 11 forms a rounded shoulder on its lower side adjacent to the screw shaft. The bone screw also includes a hexagon socket 15 (Fig. 10).

Reference is made to Figures 5, 6 and 7 which show the details of the oblong holes 4 of invention. The oblong hole 4 is made up of a first hole region 41, a second hole region 42 and a transitional region 43. The first hole region 41 is of rotational symmetric shape and its axis which is perpendicular to the body plane of the bone plate is marked at 41. The second hole region is also of rotational symmetric shape and its axis is marked at 42 in Fig. 5. The hole regions 41, 42 have such diameters and the axes of the hole regions are arranged in such a distance that the hole regions partially overlap. In a plan view, the overlapping area is characterized by two intersecting circular arcs that produce a pair of opposing tips. In the bone plate of invention, any such tips are removed, and the region where such tips are removed is the transitional region 43.

The first hole region 41 includes an upper segment 50 of cylindrical shape and a lower segment 60 of tapering shape. In the embodiment shown, a recess 7 is provided between the segments 50 and 60. The upper segment 50 carries the cylindrical thread 5 on an arc or circumferential angle α of between 120 and 180 degrees, preferably close to 179 degrees. The lower segment 60 of tapering shape may be spherical, however, a conical shape is preferred. The surface of the segment 60 is a smooth bearing surface to form the support structure 6 for a bone screw. The lower segment 60 opens to the lower side 3 of the plate 1 with an opening 61 that makes an arc or circumferential angle β of between 200 and 300 degrees, and preferably close to 260 degrees.

The second hole region 42 includes an upper segment 82 of semicircular cross section and with a smooth bearing surface to offer a support structure for the screw head shown in Fig. 9. The second hole region 42 also includes a lower section 44, preferably of cylindrical shape, but also of widening shape can be used. At the lower end of the segment 44, an opening 45 is formed which in the embodiment shown is smaller than the opening 61 formed by the lower segment 60 of the first hole region 41. See Fig. 6.

Considering the distance between the axes 41, 42 and the diameters at the openings 45 and 61, tips at the intersection of the opening circles would be formed. In the area of the lower segments 44 and 60, a flat surface 47 is machined between the segments 45 and 60 that extends parallel to the longitudinal axis of the plate body 1. By doing so, a passage is created for lateral movement for the threaded shaft 12 of the bone screw 10. In the case of Figure 6, with the flats 47 extending parallel to the longitudinal axis, the circumferential angle of the opening 45 is 180 degrees. If the flats 47 widen, seen from the opening 45, the circumferential angle is smaller, say 160 degrees. In each case, a keyhole shape is formed.

Another task for the transitional region 43 is to create a slanting or sloping guideway 80 between the upper segment 50 of the first hole region 41 and the upper segment 82 of the second hole region 42. This slanting or sloping guideway 80 is best shown in Fig. 7 and provides a smooth curved transition without any edge between the segments 82 and 50.

The oblong hole 4 contains this slanting or sloping guideway 80 at its opening facing the upper side 2 of the bone plate 1 for supporting and guiding the respective screw head 11 or 21 into the first hole region 41 by means of the inclination of the guideway surfaces 80 into the segment 60. In particular, the guideway 80 intersects the plane E_{P} of the plate at an inclination angle γ (Fig. 2). Likewise, the guideway is also inclined relative to the thread axis A_{G} by the angle δ (Fig. 2) .

Figures 10 to 13 show the cooperation between the bone screw 10 of the first traditional kind or the bone screw 20 of the second type and the bone plate 1 of the claimed system.

Figures 10 and 12 show a traditional bone screw 10 inserted into the oblong hole 4 of the bone plate 1, in a skewed position. This bone screw 10 lies with its smooth, spherically formed shoulder of the screw head 11 against the complementary formed upper segment 82 of the oblong hole 4. The threaded shaft 12 can take any skewed position which is made possible with the shape of the rounded segment 82.

Figure 13 shows inserting of the bone screw 20 into a bone for compressing bone fractures. To that end, the portion 23 is made sliding along the guideway 80. This sliding movement leads to a relative lateral displacement between screw 20 (and therefore also bone) to the bone plate 1, and a compression effect is achieved to the fractured bones. This occurs when screwing-in the bone screw 20, such that the bone plate 1 is pressed in a direction or a bone being fixed therewith is pulled in the other direction.

The guideway 80 can also be used for the traditional bone screw 10 which can become tilted or skewed relative to the vertical (corresponding to the direction of the thread axis). This gives a multitude of possibilities for securing the bone screw 10 in the bone. This tilting is possible not only in a direction along the longitudinal axis of the oblong bole, but also in directions transverse to it, so that ultimately one has a region of basically 360 degrees in which the screw can be tilted relative to the thread axis.

Such a traditional bone screw 10 can also be used with the bone plate 1 to achieve a compression effect. However, this bone screw is not suitable to achieving an angle-stable securing in bone plate 1 and bone. For this purpose one can use the bone screw 20 adapted to the bone plate as shall now be described with the help of Fig. 11.

The tapering portion 23 of the screw head 21 allows the bone screw 20 to be led into the lower segments 60. This is the peculiarity of the bone screw 20 that it can be secured in stable condition in the oblong hole 4 of the bone plate I. This situation is shown in Fig. 11. In this position, the thread 24 of the screw head 21 engages with the thread flights 5 in the oblong hole 4, and the bearing structure 23 lies with positive fit against the support structure 60. Thanks to the cooperation of the thread flights 5 with the thread 24, the lower side of the tapering portion 23 forms a bearing structure and is pressed firmly against the lower segment 60 that also forms a support structure, while it should be noted that the segment 60 and the thread flights 5 are coaxially configured, just like the thread 24 and the tapering portion 23 on the screw head 21. The transition from the screw head 21, more precisely, the thread 24 on the screw head 21, to the thread flights 5 in the oblong hole 4 is facilitated and guided by the initially occurring contact between the tapering portion 23 and the segment 60, which contact dictates a definitely guided movement. Thus, the start of the thread flight of the thread 24 can be taken securely into the thread flights 5, so that the thread 24 ultimately engages with the thread flights 5, without tilting. Furthermore, there is always pressure from the inclined surface 80 onto the screw head 21 in direction of the thread flights 5 to close any gap between the threads in engagement.

Only thanks to this fact is it possible to configure the oblong hole 4 as continuous and serviceably over its full length for either compression or for angle-stable fixation.

Although the invention herein has been described with reference to particular embodiments, it is to be understood that these embodiments are merely illustrative of the principals and applications of the present invention. Accordingly, while the invention has been described with reference to the structures and processes disclosed, it is not confined to the details set forth, but is intended to cover such, modifications or changes as may fall within the scope of the following claims.

## Claims

1. A system for fixating parts of a fractured bone including a bone plate (1) and bone screws, the bone plate adapted to cooperate with the bone screws, each having a head (11,21) and a screw shaft (12,22) and including two types (10, 20) of bone screws, the first type screw (10) having the head (11) with a rounded shoulder and the second type screw (20) having the head (21) with thread (24) on an upper cylindrical portion and a lower tapering portion (23) adjacent to the screw shaft (22)" the bone plate comprising
- a stripe-formed plate body (1) of tissue tolerance rigid material defining a body plane and a longitudinal axis and having
- a lower side (3) for resting against a fractured bone,
- an upper side (2) opposite to the lower side, and
- a plurality of oblong holes (4) extending from the upper side (2) to the lower side (3) of the plate body (1);
at least one of said oblong holes (4) comprising
- a first hole region (41),
- a second hole region (42) and
- a transitional region (43) between the first and second regions;
- said first hole region (41) being of a rotational symmetric shape with a first axis perpendicular to the body plane and comprising
- an upper segment (50) of cylindrical shape, and
- a lower segment (60) of tapering shape,
- said upper cylindrical segment (50) including a thread (5), which fits to the thread (24) on the upper cylindrical head portion of the second type screw (20) and when in engagement achieves an angle-stable securing condition in the bone plate (1),
- said lower segment (60) including a smooth surface to form a support structure (6);
- said second hole region (42) being of rotational symmetric shape with a second axis perpendicular to the body plane and in a distance to the first axis along the longitudinal axis of the plate body (1), the second hole region (42) comprising
- an upper segment (82) of semi-circular cross section and with a smooth surface to offer a support for the screw head (11) having a rounded shoulder, and
- a lower section (44) of cylindrical or widening shape for offering a passage for the screw shaft (12, 22) of both screw types (10, 20);
- said first and second hole regions (41. 42) partially overlapping at said transitional region (43);
- said transitional region (43) forming a lateral passage free of any barrier between the lower segments (44, 60) of the first and second hole regions (41, 42) for the screw shaft (12, 22) of both bone screw types (10, 20);
- said transition region (43) also forming a guideway (8, 80) sloping from the second hole region (42) to the first hole region (41) so as to allow the second type screw (20) to be inserted into the second hole region (42) and make sliding the tapering portion (23) along the guideway (8, 80) to engage the thread (24) on the upper cylindrical head portion with the thread (5) on the upper cylindrical segment (50) of the first hole region (41), and to compress bone fractures.

2. The system of claim 1
wherein said first hole region (41) of said bone plate is of a diameter larger than said second hole region (42).

3. The system of claim 2
wherein said upper segment (50) of the first hole region (41) has said thread (5) on a circumferential angle of between 120 and less than 180 degrees.

4. The system of claim 3
wherein said circumferential angle is in a range of 170 to 179 degrees

5. The system of claim 1
wherein said first hole region (41), at the lower side (3) of the plate body (1), provides for a first opening (61) having a first circular edge of a first circumferential angle (β), and
said second hole region (42), at the lower side (3) of the plate body (1), provides for a second opening (45) having a second circular edge of a second circumferential angle, one of said first and second circumferential angles exceeding 180 degrees, whereas the other of these angles not exceeding 180 degrees.

6. The system of claim 5
wherein said first circumferential angle (β) of said first hole region (41) is between 200 and 300 degrees, whereas said second circumferential angle of said second hole region is between 160 and 180 degrees.

7. The system of claim 5
wherein said first circular edge is connected to said second circular edge through straight edge portions (47) of said oblong hole (4) that extend parallel to the longitudinal axis of the plate body (1).

8. The system of claim 1
wherein said transitional region (43) includes a curved transition area between said upper segment (82) of said second hole region (42) and said upper segment (50) of said first hole region (41).

9. The system of claim 1
wherein a recess (7) is provided between the upper and lower segments (50, 60) of the first hole region (41).

10. The system of claim 1
wherein the tapering portion (23) of the screw head (21) of the second type screw (20) is a conical portion.

## Patentansprüche

1. Zur System-Fixierung von Teilen eines gebrochenen Knochens, umfassend eine Knochenplatte (A1) und Knochenschrauben, wobei die Knochenplatte zur Zusammenarbeit mit den Knochenschrauben ausgebildet ist, die jeweils einen Kopf (11,21) und einen Schraubenschaft (12,22) aufweisen und zwei Typen (10,20) von Knochenschrauben umfassen, von denen die erste Art von Knochenschraube (10) den Kopf (11) mit einer gerundeten Schulter aufweist und der zweite Typ von Knochenschraube (20) den Kopf (21) mit Schraubgewinde (24) an einem oberen zylindrischen Abschnitt besitzt und ein unterer sich verjüngender Abschnitt (23) ,benachbart dem Schraubenschaft (22), vorgesehen ist, wobei die Knochenplatte umfasst:
- einen bandförmigen Plattenkörper (1) aus gefäßtolerantem festem Material, der eine Körperebene und eine Längsachse definiert sowie
- eine untere Seite (3) zum Aufruhen gegen einen gebrochenen Knochen,
- eine obere Seite (2), entgegengesetzt zur unteren Seite und
- eine Mehrzahl von Langlöchern (4), die sich von der oberen Seite (2) zur unteren Seite (3) des Plattenkörpers (1) erstrecken;
mindestens eines der Langlöcher (4) umfasst:
- eine erste Lochregion (41),
- eine zweite Lochregion (42) und
- eine Übergangsregion (43) zwischen der ersten und der zweiten Region;
- die erste Lochregion (41) ist von drehsymmetrischer Gestalt mit einer ersten Achse senkrecht zur Körperebene und umfasst:
- einen oberen Abschnitt (50) von zylindrischer Gestalt, und
- einen unteren Abschnitt (60) von verjüngender Gestalt;
- der untere zylindrische Abschnitt (50)umfasst ein Schraubgewinde (5), welches zu dem Schraubgewinde (24) an dem oberen zylindrischen Kopfabschnitt des zweiten Typs von Knochenschraube (20) passt und im Eingriff einen winkelstabilen Befestigungszustand in der Knochenplatte (1) erzielt,
- der untere Abschnitt (60) umfasst eine glatte Oberfläche zur Bildung einer Stützstruktur (6);
- die zweite Lochregion (42) ist von rotationssymmetrischer Gestalt mit einer zweiten Achse senkrecht zur Körperebene und in einem Abstand zur ersten Achse,
entlang der Längsachse des Plattenkörpers (1), wobei die zweite Lochregion (42) umfasst;
- einen oberen Abschnitt (82) von halbkreisförmigem Querschnitt und mit einer glatten Oberfläche, um für den Schraubenkopf (11) mit der gerundeten Schulter eine Stütze zu bieten, und
- einen unteren Abschnitt (44) von zylindrischer oder sich erweiternder Gestalt, um einen Durchgang für den Schraubenschaft (12,22) beider Typen von Knochenschrauben (10,20) zu bieten;
- die ersten und die zweiten Lochregionen (21,22) überlappen sich teilweise in der Übergangsregion (43);
- die Übergangsregion (43) bildet einen seitlichen Durchgang ohne Hindernis zwischen den unteren Abschnitten (44,60) in der ersten und der zweiten Lochregion (41,42) für den Schraubenschaft (12,22) beider Typen von Knochenschrauben (10,20);
- der Übergangsabschnitt (43) bildet auch eine Führung (8,80), die von der zweiten Lochregion (42) zu der ersten Lochregion (41) hinab führt, um dem zweiten Typ von Knochenschraube (20) zu ermöglichen, in die zweite Lochregion (42) eingeführt zu werden und den sich verjüngenden Kopfabschnitt (23) entlang des Führungsweges (8,80) gleiten zu lassen, um das Schraubgewinde (24) am oberen zylindrischen Kopfabschnitt mit dem Schraubgewinde (5) auf dem oberen zylindrischen Abschnitt (50) der ersten Lochregion (41) in Eingriff treten zu lassen und die Knochenbruchteile aneinander zu pressen.

2. System nach Anspruch 1,
- worin die erste Lochregion (41) der Knochenplatte einen größeren Durchmesser aufweist als die zweite Lochregion (42).

3. System nach Anspruch 2,
- worin der obere Abschnitt (50) der ersten Lochregion (41) ein Schraubgewinde (5) auf einen Umfangswinkel zwischen 120° und weniger als 180 °aufweist.

4. System nach Anspruch 3,
- worin der Umfangswinkel im Bereich von 170° bis 179° liegt.

5. System nach Anspruch 1,
- worin die erste Lochregion (41) auf der unteren Seite (3) des Plattenkörpers (1) für eine erste Öffnung (61) mit einem ersten kreisförmigen Rand eines ersten Umfangswinkels (β) sorgt, und die zweite Lochregion (42) auf der Unterseite (3) des Plattenkörpers (1) für eine zweite Öffnung (45) mit einem zweiten kreisförmigen Rand eines zweiten Umfangswinkels sorgt, wobei eine der ersten und zweiten Umfangswinkel 180° übersteigt, während der andere dieser Winkel 180° nicht übersteigt.

6. System nach Anspruch 5,
- worin der erste Umfangswinkel (β) der ersten Lochregion (41) zwischen 200 ° und 300 ° liegt, während der zweite Umfangswinkel der zweiten Lochregion zwischen 160° und 180 ° beträgt.

7. System nach Anspruch 5,
- worin der erste kreisförmige Rand mit dem zweiten kreisförmigen Rand über einen geraden Randteil (47) des Langloches (4) verbunden ist, der sich parallel zu der Längsachse des Plattenkörpers (1) erstreckt.

8. System nach Anspruch 1,
- worin die Übergangsregion (43) einen gekrümmten Übergangsbereich zwischen dem oberen Abschnitt (82) der zweiten Lochregion (42) und dem oberen Abschnitt (50) der ersten Lochregion (41) umfasst.

9. System nach Anspruch 1,
- worin eine Aussparung (7) zwischen dem oberen und dem unteren Abschnitt (50,60) der ersten Lochregion (41) vorgesehen ist.

10. System nach Anspruch 1,
- worin der sich verjüngende Abschnitt (23) des Schraubkopfes (21) des zweiten Typs von Knochenschraube (20) konisch ausgebildet ist.

## Revendications

1. Système pour fixer des parties d'un os fracturé, comprenant des plaques vissées (1) et des vis à os, la plaque vissée étant adaptée à coopérer avec Les vis à os, chacune ayant une tête (11, 21) et une tige de vis (12, 22) et comprenant deux types (10, 20) de vis à os, la vis du premier type (10) ayant une tête (11) munie d'un épaulement arrondi et la vis du second type (20) ayant une tête (21) avec un filet (24) sur une portion cylindrique supérieure et une portion effilée inférieure (23) adjacente à la tige de vis (22), la plaque vissée comprenant :
- un corps de plaque en forme de bande (1) constitué d'une matière rigide tolérée par les tissus, définissant un plan de corps et un axe longitudinal et ayant :
- un côté inférieur (3) pour reposer contre un os fracturé,
- un côté supérieur (2) opposé au côté inférieur, et
- une pluralité de trous oblongs (4) s'étendant du côté supérieur (2) au côté inférieur (3) du corps de plaque (1) ;
au moins un desdits trous oblongs (4) comprenant :
- une première région de trou (41),
- une seconde région de trou (42) et
- une région de transition (43) entre les première et seconde régions ;
- ladite première région de trou (41) ayant une forme présentant une symétrie de rotation avec un premier axe perpendiculaire au plan du corps et comprenant :
- un segment supérieur (50) de forme cylindrique, et
- un segment inférieur (60) de forme effilée,
- ledit segment cylindrique supérieur (50) comprenant un filet (5), qui s'adapte au filet (24) sur la portion de tête cylindrique supérieure de la vis du second type (20) et, lorsqu'il est en prise, parvient à une condition de fixation à angle stable dans la plaque vissée (1),
- ledit segment inférieur (60) comprenant une surface lisse pour former une structure de support (6) ;
- ladite seconde région de trou (42) ayant une forme présentant une symétrie de rotation avec un second axe perpendiculaire au plan du corps et avec une distance par rapport au premier axe le long de l'axe longitudinal du corps de plaque (1), la seconde région de trou (42) comprenant :
- un segment supérieur (82) de section transversale semi-circulaire et avec une surface lisse pour fournir un support à la tête de vis (11) ayant un épaulement arrondi, et
- une section inférieure (44) de forme cylindrique ou s'élargissant pour fournir un passage pour la tige de vis (12,22) des deux types de vis (10, 20) ;
- lesdites première et seconde régions de trou (41, 42) se chevauchant partiellement au niveau de ladite région de transition (43) ;
- ladite région de transition (43) formant un passage latéral dépourvu de toute barrière entre les segments inférieurs (44, 60) des première et seconde régions de trou (41, 42) pour la tige de vis (12, 22) des deux types de vis (10, 20) ;
- ladite région de transition (43) formant également une voie de guidage (8, 80) s'inclinant de la seconde région de trou (42) à la première région de trou (41) de manière à permettre à la vis du second type (20) d'être insérée de la seconde région de trou (42) et de réaliser le glissement de la portion effilée (23) le long de la voie de guidage (8, 80) pour mettre en prise le filet (24) sur la portion de tête cylindrique supérieure avec le filet (5) sur le segment cylindrique supérieur (50) de la première région de trou (41), et pour comprimer des fractures osseuses.

2. Système suivant la revendication 1,
dans lequel ladite première région de trou (41) de ladite plaque vissée a un diamètre supérieur à celui de ladite seconde région de trou (42).

3. Système suivant la revendication 2,
dans lequel ledit segment supérieur (50) de la première région de trou (41) comporte ledit filet (5) sur un angle circonférentiel compris entre 120 et moins de 180 degrés.

4. Système suivant la revendication 3,
dans lequel ledit angle circonférentiel est compris dans un intervalle de 170 à 179 degrés.

5. Système suivant la revendication 1,
dans lequel ladite première région de trou (41), du côté inférieur (3) du corps de plaque (1), fournit un premier orifice (61) ayant un premier bord circulaire d'un premier angle circonférentiel (β), et
ladite seconde région de trou (42), du côté inférieur (3) du corps de plaque (1), fournit un second orifice (45) ayant un second bord circulaire d'un second angle circonférentiel, un desdits premier et second angles circonférentiels étant supérieur à 180 degrés, tandis que l'autre de ces angles ne dépasse pas 180 degrés.

6. Système suivant la revendication 5,
dans lequel ledit premier angle circonférentiel (β) de ladite première région de trou (41) est compris entre 200 et 300 degrés, tandis que ledit second angle circonférentiel de ladite seconde région de trou est compris entre 160 et 180 degrés.

7. Système suivant la revendication 5,
dans lequel ledit premier bord circulaire est connecté audit second bord circulaire par des portions de bord droit (47) dudit trou oblong (4) s'étendant parallèlement à l'axe longitudinal du corps de plaque (1).

8. Système suivant la revendication 1,
dans lequel ladite région de transition (43) comprend une zone de transition incurvée entre ledit segment supérieur (82) de ladite seconde région de trou (42) et ledit segment supérieur (50) de ladite première région de trou (41)

9. Système suivant la revendication 1,
dans lequel un évidement (7) est fourni entre les segments supérieur et inférieur (50, 60) de la première région de trou (41).

10. Système suivant la revendication 1,
dans lequel la portion effilée (23) de la tête de vis (21) de la vis du second type (20) est une portion conique.
